# EUROPÄISCHE PATENTSCHRIFT

(11) **EP 3 869 926 B1**
(45) Veröffentlichungstag und Bekanntmachung des Hinweises auf die Patenterteilung: **17.04.2024**
(21) Anmeldenummer: 21153167.8
(22) Anmeldetag: 25.01.2021
(51) Int. Cl.: H05K 3/32, A41D 1/00, A41D 1/08, A41D 13/12, A61B 5/00, H05K 1/02, H05K 1/03

(54) **TEXTILER TRÄGER**
TEXTILE SUPPORT
SUPPORT TEXTILE

(30) Priorität: 03.02.2020 AT 500842020
(43) Veröffentlichungstag der Anmeldung: 25.08.2021
(73) Patentinhaber: FH OÖ Studienbetriebs GmbH, 4600 Wels (AT)
(72) Erfinder: Langer, Josef, 4222 St.Georgen/Gusen (AT); Eibensteiner, Florian, 4180 Zwettl an der Rodl (AT); Petz, Phillip, 4040 Linz (AT)
(74) Vertreter: KLIMENT & HENHAPEL

(56) Entgegenhaltungen:
- EP-A1- 1 881 413
- WO-A1-2004/006700
- WO-A1-2017/075703
- WO-A1-2017/117048
- WO-A1-2017/156246
- DE-A1-102008 020 225
- DE-A1-102016 224 565
- US-A1- 2010 250 805
- US-A1- 2010 302 745
- US-A1- 2012 072 626

## Beschreibung

Die Erfindung betrifft ein Verfahren zur Identifizierung und Lokalisierung von auf einem textilen Träger verteilt angeordneten und zur Spannungsversorgung und Datenübertragung elektrisch miteinander verbundenen Mikroprozessormodulen, gemäß dem Oberbegriff von Anspruch 1, sowie einen textilen Träger mit einer Mehrzahl von Mikroprozessormodulen, die über den Träger verteilt angeordnet sind und zur Spannungsversorgung und Datenübertragung elektrisch miteinander verbunden sind, wobei Aufnahmen am textilen Träger vorgesehen sind, in denen jeweils ein Mikroprozessormodul austauschbar aufgenommen und mit Anschlussstellen elektrisch kontaktierbar ist, gemäß dem Oberbegriff von Anspruch 5.

Bei textilen Trägern handelt es sich um flächenförmige textile Gebilde wie Gewebe, Gewirke, Gestricke, Geflechte, Nähgewirke, Vliesstoffe oder Filze, oder um daraus gefertigte räumliche Gebilde, die aus textilen Rohstoffen wie Naturfasern oder Chemiefasern hergestellt werden. Beispiele für textile Träger sind etwa Kleidungsstücke, Heimtextilien, Taschen und vieles mehr. Dabei ist die zusätzliche Verwendung von nichttextilen Rohstoffen im Erzeugnis durchaus möglich, solange der textile Gesamtcharakter erhalten bleibt, die Fremdmaterialien also nur eine zusätzliche Funktion ausüben.

Hinsichtlich solcher textiler Träger wird zunehmend versucht sie mit prozessorgestützten, elektronischen Bauelementen wie Sensoren beispielsweise für Temperatur, Druck, Beschleunigung, Lage oder Drehung, Aktoren, die ein elektrisches Signal in mechanische Bewegungen oder Veränderungen physikalischer Größen wie Druck oder Temperatur umsetzen, oder auch Eingabeelemente oder Anzeigeeinrichtungen zu versehen, die über den textilen Träger verteilt angeordnet sind. Ein gattungsgemäßer Träger wird etwa in der DE 10 2016 224 565 A1 beschrieben. Ein weiterer textiler Träger mit Sensoren wird in der WO 2017/117048 A1 beschrieben. Der US 2010/250805 A1 und der EP 1 881 416 A1 kann grundlegendes zur Ausführung von Master-Slave-Strukturen entnommen werden. Weiterer einschlägiger Stand der Technik kann der WO 2017/156246 A1, DE 10 2008 020225 A1, US 2010/302745 A1, WO 2004/006700A1, US 2012/072626 A1 und WO 2017/075703 A1 entnommen werden.

Mit elektronischen Bauelementen ausgestattete textile Träger werden auch als "Intelligente Textilien" (smart textiles) bezeichnet. Diese prozessorgestützten, elektronischen Bauelemente sind dabei als Einheiten ausgeführt, die in weiterer Folge auch als Mikroprozessormodule bezeichnet werden, und über elektrische Leitungen, die sich über den textilen Träger erstrecken, mit Stromquellen oder mit Steuermodulen verbunden sind. In der Praxis stellt sich dabei das Problem, dass beispielsweise bei der Gewinnung und Auswertung von Daten mitunter keine örtliche Zuordnung mehr vorgenommen werden kann, also an welcher Position am textilen Träger sich das betreffende Mikroprozessormodul befindet. Diese Information ist oft erwünscht, etwa wenn ermittelt werden soll, an welcher Position des textilen Trägers beispielsweise eine bestimmte Temperatur gemessen wurde. Das Problem der korrekten örtlichen Zuordnung stellt sich insbesondere dann, wenn die Mikroprozessormodule temporär entfernt werden müssen, etwa um eine Reinigung des textilen Trägers zu ermöglichen, oder um den textilen Träger einzurollen oder zu falten. Wenn die Mikroprozessormodule anschließend wieder am textilen Träger angeordnet werden und Daten, die mit einer Kennung des jeweiligen Mikroprozessormoduls versehen sind, etwa an ein Steuermodul liefern, müsste für eine örtlich korrekte Zuordnung der Daten die Positionierung des jeweiligen Mikroprozessormoduls am textilen Träger verifiziert werden. Diese Identifizierung und Lokalisierung der auf dem textilen Träger verteilt angeordneten Mikroprozessormodule sollte vorzugsweise ohne Zutun des Anwenders erfolgen, dem es im Interesse einer einfachen Anwendbarkeit zudem auch gestattet sein sollte, die Mikroprozessormodule beliebig auf dem textilen Träger zu positionieren.

Es besteht somit das Ziel der Erfindung darin, nicht nur eine genaue Identifizierung eines jeden Mikroprozessormoduls am textilen Träger zu ermöglichen, sondern auch die Bestimmung seiner Position am textilen Träger, und zwar auch dann, wenn die Mikroprozessormodule etwa wegen einer Reinigung des Textils entfernt und in veränderter Anordnung wieder am textilen Träger positioniert werden.

Dieses Ziel wird durch die Merkmale von Anspruch 1 sowie 5 erreicht. Anspruch 1 bezieht sich auf ein Verfahren zur Identifizierung und Lokalisierung von auf einem textilen Träger verteilt angeordneten und zur Spannungsversorgung und Datenübertragung elektrisch miteinander verbundenen Mikroprozessormodulen, bei dem erfindungsgemäß vorgeschlagen wird, dass eines der Mikroprozessormodule als Mastermodul betrieben wird und die verbleibenden Mikroprozessormodule als an vorgegebenen Positionen des textilen Trägers austauschbar angeordnete und mit dem Mastermodul über einen Bus verbundene Slavemodule, wobei für jedes Slavemodul die Versorgungsspannung zwischen dem Slavemodul zu dem als elektrisch leitfähiges Garn ausgeführten Bus gemessen wird und das Mastermodul anhand der mit zunehmender Entfernung eines Slavemoduls zum Mastermodul abnehmenden Versorgungsspannung jedes Slavemodul einer der vorgegebenen Positionen auf dem textilen Träger lokalisierend zuordnet. Die Erfindung nutzt dabei die besonderen textilen Gegebenheiten durch Verwendung eines leitfähigen Garns, mit dem eine Bus-Struktur mit einer entsprechend geeigneten Bus-Enumerierung verwirklicht wird. Auf den leitfähigen Garn wird in weiterer folge noch genauer eingegangen werden, an dieser Stelle sei lediglich erwähnt, dass leitfähige Garne in unterschiedlicher Ausführung existieren, die deren Verarbeitung im Rahmen gängiger Verfahren zur Herstellung textiler Träger gestatten und elektrisch leitfähig sind. Das Ausmaß der elektrischen Leitfähigkeit variiert je nach Ausführung des Garns, der elektrische Widerstand liegt aber weit über jenem metallischer Leiter. Dieser vergleichsweise hohe Widerstand elektrisch leitfähiger Garne wird erfindungsgemäß für eine Enumerierung der Mikroprozessormodule ausgenutzt, die auch deren Lokalisierung am textilen Träger umfasst. Durch die serielle Verbindung der als Slavemodule betriebenen Mikroprozessormodule mit dem als Mastermodul betriebenen Mikroprozessormodul, die auch als "Daisychain"-Aufbau bezeichnet wird, nimmt nämlich die Versorgungsspannung zwischen einem Slavemodul zu dem als elektrisch leitfähiges Garn ausgeführten Bus mit zunehmender Entfernung eines Slavemoduls zum Mastermodul deutlich messbar ab. Werden nun erfindungsgemäß die an vorgegebenen Positionen des textilen Trägers angeordneten Slavemodule über einen Bus seriell verbunden, können die vorgegebenen Positionen über den jeweils abnehmenden Wert der Versorgungsspannung unterschieden werden, wodurch eine Lokalisierung der Slavemodule möglich ist.

Konkret kann dabei etwa so vorgegangen werden, dass eine Identifizierungsanfrage des Mastermoduls an die Slavemodule erfolgt, mit der das Mastermodul einer von einem Slavemodul gemessenen Versorgungsspannung eine das jeweilige Slavemodul identifizierende Identifizierungsinformation zuordnet. Die Identifizierungsinformation ist in einem Speicher des jeweiligen Slavemoduls gespeichert und ändert sich nicht. Hingegen ändert sich die Versorgungsspannung eines Slavemoduls zum Bus nach Entnahme und Neuanordnung des Slavemoduls an einer anderen Position der vorgegebenen Positionen am textilen Träger. Daher wird gemäß einer Ausführungsform vorgeschlagen, dass die Slavemodule nach Neuanordnung der Slavemodule an den vorgegebenen Positionen jeweils die Versorgungsspannung zum Bus messen und in einem Speicher des jeweiligen Slavemoduls gemeinsam mit der Identifizierungsinformation des jeweiligen Slavemoduls abspeichern, wobei die Identifizierungsanfrage des Mastermoduls einen vorgegebenen Spannungsbereich enthält und nach Eingang der Identifizierungsanfrage jedes Slavemodul die in seinem Speicher gespeicherte Versorgungsspannung mit dem vorgegebenen Spannungsbereich der Identifizierungsanfrage vergleicht und ein Slavemodul seine Identifizierungsinformation an das Mastermodul übermittelt, falls die gespeicherte Versorgungsspannung des betreffenden Slavemoduls innerhalb des vorgegebenen Spannungsbereiches der Identifizierungsanfrage liegt. Durch schrittweise Erhöhung oder Verringerung des abgefragten Spannungsbereiches kann das Mastermodul schließlich eine Reihung der Slavemodule und eine Zuordnung zu den bekannten, vorgegebenen Positionen am textilen Träger vornehmen.

Freilich könnte die Identifizierungsanfrage des Mastermoduls alternativ auch die Aufforderung zur Übersendung der gemessenen Versorgungsspannung gemeinsam mit der Identifizierungsinformation umfassen, allerdings müssten in diesem Fall die für einen Bus erforderlichen Vorkehrungen für einen geregelten Kommunikationsablauf getroffen werden.

Die Durchführung des erfindungsgemäßen Verfahrens wird in der Regel nur anfänglich erforderlich sein, etwa wenn die Mikroprozessormodule entfernt und neu am textilen Träger angeordnet wurden. Sobald die Mikroprozessormodule identifiziert und lokalisiert wurden, können die Mikroprozessormodule entsprechend ihrer vorgesehenen Funktion betrieben werden. Daher wird auch vorgeschlagen, dass die Identifizierungsanfrage des Mastermoduls an die Slavemodule in einer anfänglichen Enumerationsphase nach Neuanordnung der Slavemodule an den vorgegebenen Positionen erfolgt, und nach der Identifizierung und Lokalisierung der Slavemodule in einer nachfolgenden Betriebsphase die am textilen Träger örtlich lokalisierbare Übertragung von Daten zu Steuerungs-, Auswertungs- oder Anzeigezwecken zwischen den Slavemodulen und dem Mastermodul erfolgt.

Die Erfindung bezieht sich des Weiteren auf einen textilen Träger mit einer Mehrzahl von Mikroprozessormodulen, die über den Träger verteilt angeordnet sind und zur Spannungsversorgung und Datenübertragung elektrisch miteinander verbunden sind, wobei Aufnahmen am textilen Träger vorgesehen sind, in denen jeweils ein Mikroprozessormodul austauschbar aufgenommen und mit Anschlussstellen elektrisch kontaktierbar ist. Hierbei wird erfindungsgemäß vorgeschlagen, dass eines der Mikroprozessormodule als Mastermodul ausgebildet ist und die verbleibenden Mikroprozessormodule als mit dem Mastermodul über einen Bus verbundene Slavemodule, wobei jeweils ein Slavemodul in den Aufnahmen am textilen Träger austauschbar aufgenommen und mit den Anschlussstellen zum Bus elektrisch kontaktierbar ist, und der Bus als ein das Mastermodul mit den Aufnahmen verbindendes und im textilen Träger verlaufendes, elektrisch leitfähiges Garn ausgeführt ist. Ein Garn ist ein Sammelbegriff für alle linienförmigen textilen Gebilde. Danach ist ein Garn sinngemäß ein langes, dünnes Gebilde aus einer oder mehreren Fasern. Es bezeichnet in der Regel ein textiles Zwischenprodukt, das zu Geweben, Gestricken, Gewirken und Stickereien verarbeitet werden kann. Ein elektrisch leitfähiges Garn wird in der Regel durch eine Kombination von elektrisch leitenden Fasern aus einem metallischen Material mit elektrisch nichtleitenden Fasern aus natürlichen oder synthetischen Stoffen hergestellt. Je nach Metallgehalt besitzen diese Garne mehr oder weniger textile oder metallische Eigenschaften. Bekannt sind etwa Verfahren, bei denen zentral geführte Metalldrähte einfach oder doppelt textil umwunden werden. Da in diesen Garnen im Wesentlichen der Draht die Reißfestigkeit bestimmt, werden meist relativ dicke Drähte mit Durchmessern größer 0,1 mm eingesetzt. Des Weiteren ist es bekannt, wie durch die Umwindung von parallel geführtem Draht und textilem Faden auch dünne Drähte im Kern eines umwundenen Garnes eingesetzt werden können. In dieser Anordnung sorgt der zentrale textile Faden für Reißfestigkeit, während der parallel laufende dünne Draht die elektrische Leitfähigkeit des Garns bewirkt. Wie bereits ausgeführt wurde, gestatten leitfähige Garne die Verarbeitung mit textilen Trägern, wobei der elektrische Widerstand aber weit über jenem metallischer Leiter liegt. Dieser vergleichsweise hohe Widerstand elektrisch leitfähiger Garne wird erfindungsgemäß wie bereits beschrieben für eine Lokalisierung der Mikroprozessormodule am textilen Träger ausgenutzt, indem mithilfe des leitfähigen Garns eine Bus-Struktur mit einer entsprechend geeigneten Bus-Enumerierung verwirklicht wird. Hierfür sind erfindungsgemäß Aufnahmen am textilen Träger vorgesehen, in denen jeweils ein Slavemodul austauschbar aufgenommen und mit Anschlussstellen zum Bus elektrisch kontaktierbar ist. Zur Messung der Versorgungsspannung können die Slavemodule etwa mit Analog/Digital-Wandler (A/D-Wandler, ADC) versehen sein, die die anliegende Versorgungsspannung mit einer Referenzspannung vergleichen und in eine Digitalzahl umwandeln, die das Verhältnis der Messspannung zu dieser Referenzspannung ausdrückt.

Das Mastermodul und die Slavemodule können hardwaremäßig identisch ausgeführt sein, allerdings benötigt das Mastermodul eine andere Firmware als die Slavemodule, die vorzugsweise auch parametrierbar ausgeführt und über Dip-Schalter oder Pins einstellbar ist.

Insbesondere wird vorgeschlagen, dass die Aufnahmen als Aufnahmetaschen und die Slavemodule als flächige Gebilde ausgeführt sind, wobei ein Slavemodul in eine Aufnahmetasche entlang einer Einschieberichtung einschiebbar und in der Aufnahmetasche lösbar befestigbar ist. Das Slavemodul stellt ein im Wesentlichen flächiges Gebilde dar, indem seine Längserstreckung und seine Breitenerstreckung jeweils wesentlich größer sind als seine senkrecht dazu gemessene Höhenerstreckung. Die Slavemodule weisen etwa eine Platine auf, auf der die elektronischen Schaltungen sowie die elektronischen Bauelemente wie Sensoren, Aktoren, Eingabeelemente, Anzeigeeinrichtungen und dergleichen angeordnet sind. Für deren elektrische Kontaktierung wäre es grundsätzlich möglich, dass der Bus eine erste elektrische Leitung zur Spannungsversorgung sowie zur Datenübertragung umfasst, sowie eine zweite elektrische Leitung als Masse. Vorzugsweise wird jedoch vorgeschlagen, dass der Bus eine erste elektrische Leitung zur Spannungsversorgung und eine zweite elektrische Leitung als Masse umfasst, sowie eine dritte elektrische Leitung für den Empfang von Daten und eine vierte elektrische Leitung für das Senden von Daten. Die Spannungsversorgung wird dabei vom Mastermodul sichergestellt.

Für die Herstellung der elektrischen Anschlussstellen dieser vier elektrischen Leitungen zum jeweiligen Slavemodul wird gemäß einer ersten Ausführungsvariante vorgeschlagen, dass für die vier elektrischen Leitungen vier Druckknöpfe mit einander jeweils zugeordneten Druckknopfteilen an jeder Aufnahmetasche und an jedem Slavemodul für die lösbare Befestigung und als Anschlussstellen zum Bus vorgesehen sind, deren Anordnung an jeder Aufnahmetasche ausschließlich bei Drehungen des Slavemoduls um 0° oder 360° um eine zum einzuschiebenden Slavemodul senkrechte Achse eine aneinander liegende Lage der Druckknopfteile ermöglicht. Mit einer zum Slavemodul senkrechten Achse ist dabei eine Achse senkrecht zu seiner Längs- und Breitenerstreckung gemeint. Diese Maßnahmen gewährleisten eine verpolungssichere Befestigung der Slavemodule in den Aufnahmetaschen, da sie nur in einer einzigen Orientierung in die Aufnahmetaschen einschiebbar und befestigbar sind. In einer anderen Orientierung würden die einander jeweils zugeordneten Druckknopfteile der Aufnahmetasche und des Slavemoduls nämlich nicht aneinander zu liegen kommen und somit keine Befestigung erlauben. Die vier am oder im textilen Träger verlaufenden Leitungen kontaktieren dabei die an der Aufnahmetasche vorgesehenen Druckknopfteile, über die der elektrische Kontakt zum eingeschobenen und über die Druckknopfteile des Slavemoduls befestigten Slavemoduls hergestellt wird.

Alternativ wäre es aber auch möglich, dass die Anschlussstellen zum jeweiligen Slavemodul als elektrisch leitende Anschlussflächen ausgeführt sind, die auf einander zugewandten Innenflächen der Aufnahmetaschen angeordnet sind und jeweils an der Oberfläche der Slavemodule vorgesehene Anschlussflächen eines eingeschobenen Slavemoduls durch Aneinanderliegen elektrisch kontaktieren. Insbesondere kann dabei vorgesehen sein, dass die Anschlussflächen an den einander zugewandten Innenflächen einer Aufnahmetasche bei fehlendem Slavemodul als durch Aneinanderliegen miteinander kontaktierbare Flächen ausgeführt sind. Diese Maßnahme ermöglicht die Herstellung eines überbrückenden Kurzschlusses in einer Aufnahmetasche, wenn kein Slavemodul eingeschoben wird. Die Leitungen zur Spannungsversorgung, die Masseleitung, sowie die Leitungen zur Datenübertragung vom Mastermodul zu den Slavemodulen sowie von den Slavemodulen zum Mastermodul werden somit gewissermaßen "durchgeschliffen" und die Aufnahmetasche somit überbrückt.

Um auch für diese Ausführungsform eine verpolungssichere Befestigung der Slavemodule in den Aufnahmetaschen zu ermöglichen wird vorgeschlagen, dass die Anschlussflächen der Aufnahmetaschen parallel zueinander und senkrecht zur Einschieberichtung der jeweiligen Aufnahmetasche verlaufen und jeweils parallel zueinander an der Oberfläche der Slavemodule verlaufenden Anschlussflächen der Slavemodule zugeordnet sind, deren Anordnung ausschließlich bei Drehungen des einzuschiebenden Slavemoduls um 0° oder 180° um eine zum Slavemodul senkrechte Achse eine aneinander liegende Lage der Anschlussflächen der Aufnahmetasche und des eingeschobenen Slavemoduls ermöglicht. Mit einer zum Slavemodul senkrechten Achse ist dabei wiederum eine Achse senkrecht zu seiner Längs- und Breitenerstreckung gemeint. Falls die Aufnahmetaschen beispielsweise rechteckförmig ausgeführt sind und die Slavemodule als entsprechend rechteckförmig ausgeführte, flächige Gebilde, gibt es bei Drehungen um eine zum Slavemodul senkrechte Achse an sich noch zwei mögliche Orientierungen, mit denen ein Slavemodul in eine Aufnahmetasche eingeschoben werden kann. Die beschriebene Anordnung der Anschlussflächen stellt sicher, dass eine verpolungssichere Befestigung des Slavemoduls in beiden Orientierungen erfolgt, da unabhängig von der gewählten Orientierung stets eine aneinander liegende Lage der Anschlussflächen der Aufnahmetasche und des eingeschobenen Slavemoduls sichergestellt wird. Beispielsweise können die Anschlussflächen für die Versorgungsspannung als zwei äußerste Anschlussflächen ausgeführt werden, die Anschlussflächen für die Masseleitungen als zwei weiter innen liegende Anschlussflächen, und die Anschlussflächen für die dritte und vierte Leitung als eine zentral verlaufende Anschlussfläche. Somit werden bei jeder der beiden oben genannten Orientierungen stets Anschlussflächen für die Versorgungsspannung, Anschlussflächen für die Masseleitungen und Anschlussflächen für die dritte und vierte Leitung verpolungssicher kontaktiert.

Die Erfindung wird in weiterer Folge anhand von Ausführungsbeispielen mithilfe der beiliegenden Zeichnungen näher erläutert. Es zeigen hierbei die
Fig. 1 eine schematische Darstellung einer ersten Ausführungsform eines als Kleidungsstück ausgeführten textilen Trägers gemäß der Erfindung,
Fig. 2 ein schematisches Schaltungsbild für eine mögliche Ausführungsform des erfindungsgemäßen Bus-Aufbaus mit einem Mastermodul und einer Mehrzahl von Slavemodulen,
Fig. 3 eine schematische Darstellung für eine mögliche Ausführungsform der erfindungsgemäßen Bus-Leitungen zwischen dem Mastermodul und einem Slavemodul sowie zwischen zwei Slavemodulen,
Fig. 4 eine schematische Darstellung einer ersten Ausführungsform eines Slavemoduls zur Aufnahme in mit Druckknöpfen als Anschlussstellen versehenen Aufnahmetaschen,
Fig. 5 eine schematische Darstellung einer zweiten Ausführungsform einer mit Anschlussflächen als Anschlussstellen versehenen Aufnahmetasche ohne eingeschobenes Slavemodul,
Fig. 6 eine schematische Darstellung der zweiten Ausführungsform einer mit Anschlussflächen als Anschlussstellen versehenen Aufnahmetasche mit eingeschobenem Slavemodul, und die
Fig. 7 eine Explosionsdarstellung des Aufbaus der Fig. 6, bei der die Anschlussflächen des Slavemoduls ersichtlich sind.

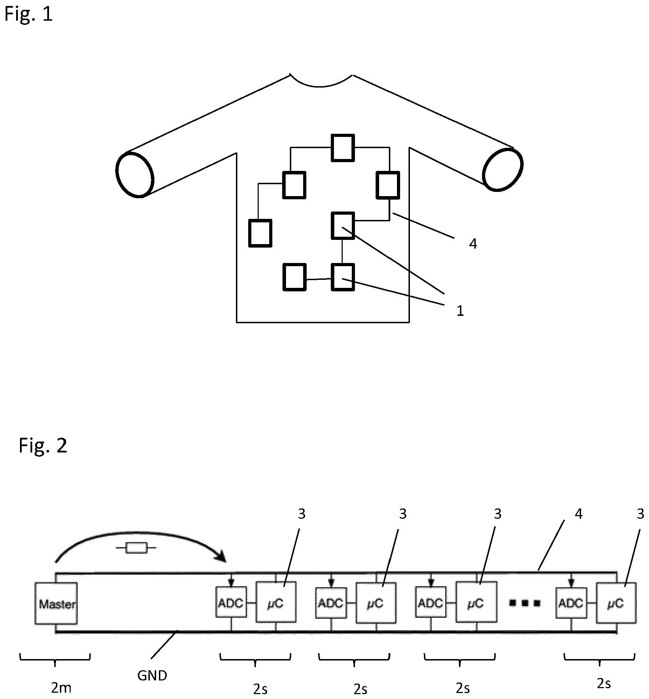

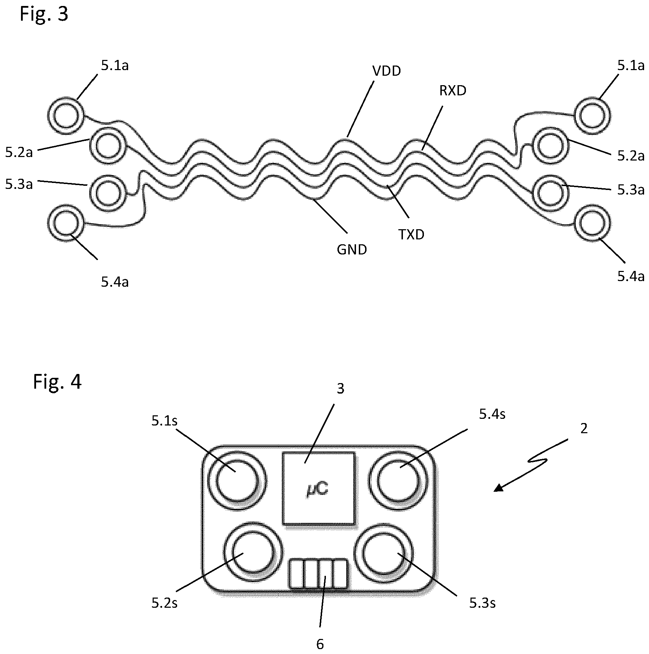

Zunächst wird auf die Fig. 1 Bezug genommen, die eine schematische Darstellung einer ersten Ausführungsform eines als Kleidungsstück ausgeführten textilen Trägers zeigt. Am textilen Träger sind eine Mehrzahl von Aufnahmetaschen 1 vorgesehen, die über die Oberfläche des textilen Trägers verteilt angeordnet sind. In den Aufnahmetaschen 1 ist jeweils ein Mikroprozessormodul 2 austauschbar aufgenommen, indem die Mikroprozessormodule 2 jeweils als flächige Gebilde ausgeführt sind, die entlang einer Einschieberichtung beispielsweise von oben nach unten in eine Aufnahmetasche 1 eingeschoben und entgegen der Einschieberichtung auch wieder den Aufnahmetaschen 1 entnommen werden können. Die Mikroprozessormodule 2 weisen jeweils einen Mikroprozessor 3 auf, sowie prozessorgestützte, elektronische Bauelemente 6 wie Sensoren beispielsweise für Temperatur, Druck, Beschleunigung, Lage oder Drehung, Aktoren, die ein elektrisches Signal in mechanische Bewegungen oder Veränderungen physikalischer Größen wie Druck oder Temperatur umsetzen, oder auch Eingabeelemente oder Anzeigeeinrichtungen (siehe auch Fig. 4). Die Mikroprozessormodule 2 werden in der Regel in herkömmlicher Weise mithilfe einer Platine gefertigt sein, auf die die genannten Komponenten angeordnet sind.

Mithilfe der Mikroprozessormodule 2 werden beispielsweise Daten wie etwa eine Temperatur gemessen. Dabei können unterschiedlich positionierte Mikroprozessormodule 2 unterschiedliche Daten liefern, wobei es mitunter wünschenswert ist, dass die Information, welches Mikroprozessormodul 2 welche Daten an welcher Position ermittelt hat, im Zuge der Auswertung der Daten nicht verloren geht. In der Praxis stellt sich dabei das Problem, dass sich eine örtliche Zuordnung der Mikroprozessormodule 2 mitunter auch verändern kann, etwa wenn die Mikroprozessormodule 2 beispielsweise im Zuge einer Reinigung des textilen Trägers temporär entfernt werden und in weiterer Folge in veränderter Zuordnung wieder in den Aufnahmetaschen 1 platziert werden. Wenn die Mikroprozessormodule 2 anschließend wieder Daten, die mit einer Kennung des jeweiligen Mikroprozessormoduls 2 versehen sind, etwa an ein Steuermodul liefern, müsste für eine örtlich korrekte Zuordnung der Daten die Positionierung des jeweiligen Mikroprozessormoduls 2 am textilen Träger verifiziert werden. Diese Identifizierung und Lokalisierung der auf dem textilen Träger verteilt angeordneten Mikroprozessormodule 2 sollte wie bereits erwähnt wurde vorzugsweise ohne Zutun des Anwenders erfolgen, dem es im Interesse einer einfachen Anwendbarkeit zudem auch gestattet sein sollte, die Mikroprozessormodule 2 beliebig auf dem textilen Träger zu positionieren.

Hierfür wird ein Bus-Aufbau vorgeschlagen, bei dem eines der Mikroprozessormodule 2 als Mastermodul 2m betrieben wird und die verbleibenden Mikroprozessormodule 2 als Slavemodule 2s, die über einen Bus 4 miteinander und mit dem Mastermodul 2m seriell verbunden sind. Eine solche Anordnung, wie sie in der Fig. 1 schematisch gezeigt ist, wird auch als "Daisychain"-Aufbau bezeichnet. Der Bus 4 wird mithilfe eines elektrisch leitfähigen Garns realisiert, der im oder am textilen Träger verläuft und jede Aufnahmetasche 1 passiert. Der vergleichsweise hohe elektrische Widerstand des elektrisch leitfähigen Garns bringt es mit sich, dass die Versorgungsspannung zwischen einem Slavemodul 2s zum Bus 4 mit zunehmender Entfernung eines Slavemoduls 2s zum Mastermodul 2m abnimmt. Diese Abnahme ist gut messbar und kann für eine Unterscheidung der einzelnen Slavemodule 2s herangezogen werden, indem das Mastermodul 2m anhand der mit zunehmender Entfernung eines Slavemoduls 2s zum Mastermodul 2m abnehmenden Versorgungsspannung jedes Slavemodul 2s einer der vorgegebenen Positionen auf dem textilen Träger lokalisierend zuordnet. Die Messung der Versorgungsspannung kann etwa mithilfe eines ADC erfolgen, mit dem jedes Slavemodul 2s ausgestattet ist.

Die Fig. 2 zeigt in schematischer Weise ein entsprechendes Schaltungsbild für eine mögliche Ausführungsform des erfindungsgemäßen Bus-Aufbaus mit einem Mastermodul 2m und einer Mehrzahl von Slavemodulen 2s, bei dem die Slavemodule 2s über den Bus 4 miteinander und mit dem Mastermodul 2m seriell verbunden sind. Die in der Fig. 2 gezeigte, untere Leitung stellt dabei eine Masseleitung für den Bus 4 dar.

Die Fig. 3 zeigt eine schematische Darstellung einer möglichen Ausführungsform für den erfindungsgemäßen Bus 4 zwischen dem Mastermodul 2m und einem Slavemodul 2s sowie zwischen zwei Slavemodulen 2s, bei der der Bus 4 eine erste elektrische Leitung VDD zur Spannungsversorgung und eine zweite elektrische Leitung GND als Masse umfasst, sowie eine dritte elektrische Leitung RXD für den Empfang von Daten und eine vierte elektrische Leitung TXD für das Senden von Daten. Die Spannungsversorgung für die Slavemodule 2s wird dabei vom Mastermodul 2m sichergestellt. Diese vier elektrischen Leitungen VDD, GND, RXD, TXD erstrecken sich jeweils zwischen zwei Anschlussstellen 5a, die jeweils im Bereich zweier Aufnahmetaschen 1 angeordnet sind. In der Fig. 3 sind hierfür die im Bereich zweier Aufnahmetaschen 1 angeordneten Anschlussstellen 5.1a, 5.2a, 5.3a und 5.4a ersichtlich.

Die Fig. 4 zeigt die den Anschlussstellen 5.1a, 5.2a, 5.3a, 5.4a einer Aufnahmetasche 1 jeweils zugeordneten Anschlussstellen 5.1s, 5.2s, 5.3s, 5.4s an den Slavemodulen 2 gemäß einer ersten Ausführungsform. Das Slavemodul 2 weist ferner einen Mikroprozessor 3 sowie ein elektronisches Bauelement 6 wie beispielsweise einen Sensor auf, die auf einer Platine angeordnet sind. Für die Herstellung des elektrischen Kontakts zwischen den vier elektrischen Leitungen VDD, GND, RXD, TXD und einem Slavemodul 2 sind gemäß der Ausführungsform der Fig. 4 vier Druckknöpfe mit einander jeweils zugeordneten Druckknopfteilen an jeder Aufnahmetasche 1 und an jedem Slavemodul 2 vorgesehen, mit denen ein Slavemodul 2 in einer Aufnahmetasche 1 lösbar befestigt und mit dem Bus 4 kontaktiert werden kann. Die Druckknöpfe sind in der gezeigten Ausführungsform so angeordnet, dass an jeder Aufnahmetasche 1 bei Drehungen des einzuschiebenden Slavemoduls 2 um ausschließlich 0° oder 360° um eine zum Slavemodul 2 senkrechte Achse eine aneinander liegende Lage der einander jeweils zugeordneten Druckknopfteile ermöglicht wird. Diese Maßnahme gewährleistet eine verpolungssichere Befestigung der Slavemodule 2 in den Aufnahmetaschen 1, da sie nur in einer einzigen Orientierung in die Aufnahmetaschen 1 einschiebbar und befestigbar sind.

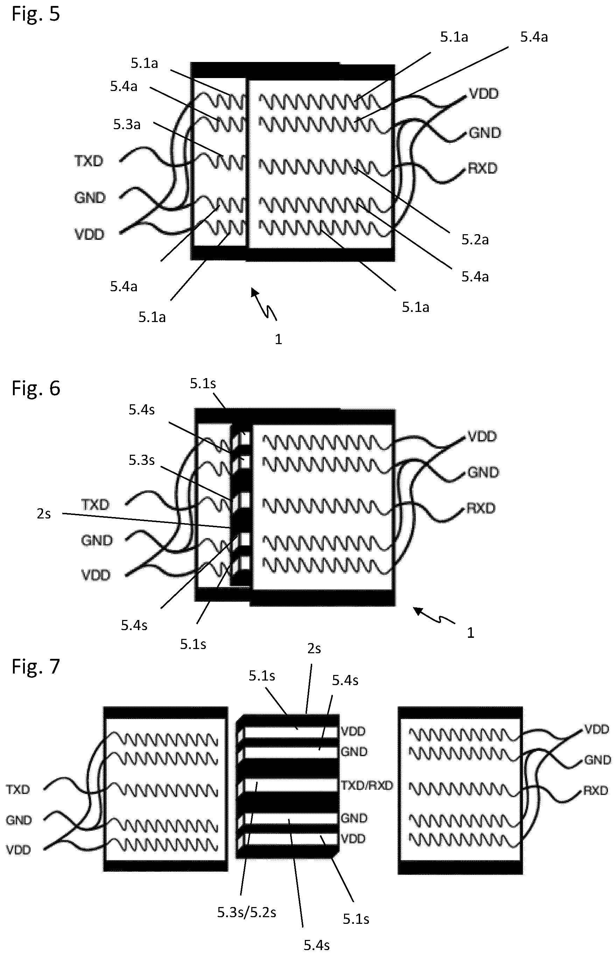

Die Fig. 5-7 zeigen eine alternative Ausführungsform, bei der die Anschlussstellen 5 zum Bus 4 als elektrisch leitende Anschlussflächen ausgeführt sind, die auf einander zugewandten Innenflächen der Aufnahmetaschen 1 angeordnet sind und jeweils an der Oberfläche der Slavemodule 2 vorgesehene Anschlussflächen eines eingeschobenen Slavemoduls 2 durch Aneinanderliegen elektrisch kontaktieren. Die Anschlussflächen können etwa so ausgeführt sein, dass sie parallel zueinander und senkrecht zur Einschieberichtung der jeweiligen Aufnahmetasche 1 verlaufen, wie in den Fig. 5-7 ersichtlich ist, wobei sie jeweils parallel zueinander an der Oberfläche der Slavemodule 2 verlaufenden Anschlussflächen der Slavemodule 2 zugeordnet sind. Die Anschlussflächen der Aufnahmetaschen 1 sind in den Fig. 5-7 durch die mäanderförmig verlaufenden Anschlussenden der jeweiligen elektrischen Leitungen angedeutet, die die jeweiligen Anschlussflächen definieren. Zudem sind die Anschlussflächen so angeordnet, dass sie bei Drehungen des Slavemoduls 2 um ausschließlich 0° oder 180° um eine zum Slavemodul 2 senkrechte Achse eine aneinander liegende Lage der Anschlussflächen der Aufnahmetasche 1 und des eingeschobenen Slavemoduls 2 ermöglichen. Mit anderen Worten sind sie um eine zur Einschieberichtung senkrechte Achse symmetrisch angeordnet. Falls die Aufnahmetaschen 1 beispielsweise rechteckförmig ausgeführt sind und die Slavemodule 2 als entsprechend rechteckförmig ausgeführte, flächige Gebilde, gibt es bei Drehungen um eine zum Slavemodul 2 senkrechte Achse an sich noch zwei mögliche Orientierungen, mit denen ein Slavemodul 2 in eine Aufnahmetasche 1 eingeschoben werden kann. Die beschriebene Anordnung der Anschlussflächen stellt sicher, dass eine verpolungssichere Befestigung des Slavemoduls 2 in beiden Orientierungen erfolgt, da unabhängig von der gewählten Orientierung stets eine aneinander liegende Lage der Anschlussflächen der Aufnahmetasche 1 und des eingeschobenen Slavemoduls 2 sicher gestellt wird. Beispielsweise können, wie in den Fig. 5-7 dargestellt ist, die Anschlussflächen für die Versorgungsspannung als zwei äußerste Anschlussflächen ausgeführt werden, die Anschlussflächen für die Masseleitungen als zwei weiter innen liegende Anschlussflächen, und die Anschlussflächen für die dritte und vierte Leitung als eine zentral verlaufende Anschlussfläche. Somit werden bei jeder der beiden oben genannte Orientierungen stets Anschlussflächen für die Versorgungsspannung, Anschlussflächen für die Masseleitungen und Anschlussflächen für die dritte und vierte Leitung verpolungssicher kontaktiert.

Wie den Fig. 5-7 ferner entnommen werden kann, sind die Anschlussflächen an den einander zugewandten Innenflächen einer Aufnahmetasche 1 bei fehlendem Slavemodul 2 (wie beispielsweise in der Fig. 5) als durch Aneinanderliegen miteinander kontaktierbare Flächen ausgeführt. Diese Maßnahme ermöglicht die Herstellung eines überbrückenden Kurzschlusses in einer Aufnahmetasche 1, wenn kein Slavemodul 2 eingeschoben wird. Die Leitungen zur Spannungsversorgung, die Masseleitung, sowie die Leitungen zur Datenübertragung vom Mastermodul 2m zu den Slavemodulen 2s sowie von den Slavemodulen 2s zum Mastermodul 2m werden somit gewissermaßen "durchgeschliffen" und die Aufnahmetasche 1 somit überbrückt.

In der praktischen Anwendung messen die Slavemodule 2 nach Neuanordnung der Slavemodule 2 an den vorgegebenen Positionen jeweils die Versorgungsspannung zum Bus 4 mithilfe ihres ADC und speichern den gemessenen Wert in einem Speicher des jeweiligen Slavemoduls 2, wo auch eine Identifizierungsinformation für das jeweilige Slavemodul 2 abgespeichert ist. Diese Messung kann entweder selbsttätig nach bestimmten Ereignissen wie beispielsweise Einschaltvorgängen, oder auch in vorgegebenen Zeitabständen erfolgen. Diese Messungen können aber auch vom Mastermodul 2m veranlasst werden, indem eine entsprechende Aufforderung an die Slavemodule 2s über den Bus 4 gesendet wird. Auch diese Aufforderung kann nach bestimmten Ereignissen wie beispielsweise Einschaltvorgängen, oder auch in vorgegebenen Zeitabständen erfolgen.

In einer anfänglichen Enumerationsphase sendet das Mastermodul 2m in weiterer Folge eine Identifizierungsanfrage an die Slavemodule 2s, die einen vorgegebenen Spannungsbereich enthält. Nach Empfang der Identifizierungsanfrage vergleicht jedes Slavemodul 2s die in seinem Speicher gespeicherte Versorgungsspannung mit dem vorgegebenen Spannungsbereich der Identifizierungsanfrage und übermittelt seine Identifizierungsinformation an das Mastermodul 2m, falls die gespeicherte Versorgungsspannung des betreffenden Slavemoduls 2s innerhalb des vorgegebenen Spannungsbereiches der Identifizierungsanfrage liegt. Durch schrittweise Erhöhung oder Verringerung des abgefragten Spannungsbereiches kann das Mastermodul 2m schließlich eine Reihung der Slavemodule 2s und eine Zuordnung zu den bekannten, vorgegebenen Positionen am textilen Träger vornehmen, da die Positionen der Aufnahmetaschen 1 und der Spannungsabfall entlang des Bus 4 an sich bekannt sind. Der vorgegebene und abgefragte Spannungsbereich ist dabei ausreichend eng zu wählen, um eine eindeutige Zuordnung zu ermöglichen. Das Mastermodul 2m speichert diese Zuordnung der Identifizierungsinformation eines Slavemoduls 2s zu der das Slavemodul 2s aufnehmenden Aufnahmetasche 1 für die folgende Betriebsphase.

In der Betriebsphase können die Slavemodule 2s entsprechend ihrer vorgesehenen Funktion betrieben werden. Sie messen beispielsweise die jeweils an ihrer Position vorliegende Temperatur und/oder andere Werte und bereiten die zu übertragenden Daten zu Mitteilungen in Form von Datenpaketen vor, die gemeinsam mit der entsprechenden Identifizierungsinformation in ein Senderegister eingestellt werden, sobald die Daten vollständig zur Übertragung vorbereitet sind. Diesen Mitteilungen kann auch ein Header für den Mitteilungsrahmen beigefügt sein, der Auskunft darüber gibt, dass es sich um Daten für das Mastermodul 2m handelt. Die Slavemodule 2s können des Weiteren dazu eingerichtet sein, ein Trigger-Signal an das Mastermodul 2m zu übermitteln, wenn durch das betreffende Slavemodul 2s Daten zur Übertragung anstehen. Das Mastermodul 2m ruft in weiterer Folge die vorbereiteten Datenpakete aus dem Senderegister des betreffenden Slavemoduls 2s ab und ordnet sie mithilfe der in der Enumerationsphase gewonnenen Zuordnung der entsprechenden Position der Aufnahmetasche 1 zu. Die so gewonnenen und lokalisierten Daten können in weiterer Folge in einem Speicher des Mastermoduls 2m für einen späteren Abruf gespeichert werden, oder sie werden über eine Schnittstelle an ein externes Gerät wie beispielsweise ein mobiles Endgerät ("Smartphone") zur weiteren Auswertung gesendet.

Alternativ können die Daten von den Slavemodulen 2s auch in vorgegebenen Zeitabständen an das Mastermodul 2m übermittelt werden. Hierbei wird in üblicher Weise ein Übertragungsprotokoll mit entsprechenden Prioritätsebenen vorgesehen sein, das die Hierarchie der Datenübertragung regelt. In der Ausführungsform der Fig. 5-7 kann etwa vorgesehen sein, dass ein Slavemodul 2s über die dritte elektrische Leitung RXD ein Datenpaket von einem anderen Slavemodul 2s erhält und dem Header dabei entnimmt, dass es sich dabei um Daten für das Mastermodul 2m handelt. Das empfangende Slavemodul 2s kann in weiterer Folge zur Übermittlung bereitstehende und mit seiner Identifizierungsinformation versehene Datenpakte dem empfangenen Datenpaket "anhängen" und sie über die vierte elektrische Leitung TXD an das benachbarte Slavemodul 2s senden. Dieser Vorgang wiederholt sich in jedem Slavemodul 2s, bis ein dem Mastermodul 2m nächstliegendes Slavemodul 2s den "Datenstring" an das Mastermodul 2m übermittelt. Das Mastermodul 2m "zerlegt" die mit den jeweiligen Identifizierungsinformationen versehenen Datenpakte und ordnet jedes Datenpaket mithilfe der in der Enumerationsphase gewonnenen Zuordnung der entsprechenden Position der Aufnahmetasche 1 zu.

Mithilfe der Erfindung gelingt es somit nicht nur eine genaue Identifizierung eines jeden Mikroprozessormoduls 2 am textilen Träger zu ermöglichen, sondern auch die Bestimmung seiner Position am textilen Träger, und zwar auch dann, wenn die Mikroprozessormodule 2 etwa wegen einer Reinigung des Textils entfernt und in veränderter Anordnung wieder am textilen Träger positioniert werden.

## Patentansprüche

1. Verfahren zur Identifizierung und Lokalisierung von auf einem textilen Träger verteilt angeordneten und zur Spannungsversorgung und Datenübertragung elektrisch miteinander verbundenen Mikroprozessormodulen (2), **dadurch gekennzeichnet, dass** eines der Mikroprozessormodule (2) als Mastermodul (2m) betrieben wird und die verbleibenden Mikroprozessormodule (2) als an vorgegebenen Positionen des textilen Trägers austauschbar angeordnete und mit dem Mastermodul (2m) über einen Bus (4) verbundene Slavemodule (2s), wobei für jedes Slavemodul (2s) die Versorgungsspannung zwischen dem Slavemodul (2s) zu dem als elektrisch leitfähiges Garn ausgeführten Bus (4) gemessen wird und das Mastermodul (2m) anhand der mit zunehmender Entfernung eines Slavemoduls (2s) zum Mastermodul (2m) abnehmenden Versorgungsspannung jedes Slavemodul (2s) einer der vorgegebenen Positionen auf dem textilen Träger lokalisierend zuordnet.

2. Verfahren nach Anspruch 1, **dadurch gekennzeichnet, dass** eine Identifizierungsanfrage des Mastermoduls (2m) an die Slavemodule (2s) erfolgt, mit der das Mastermodul (2m) einer von einem Slavemodul (2s) gemessenen Versorgungsspannung eine das jeweilige Slavemodul (2s) identifizierende Identifizierungsinformation zuordnet.

3. Verfahren nach Anspruch 2, **dadurch gekennzeichnet, dass** die Slavemodule (2s) nach Neuanordnung der Slavemodule (2s) an den vorgegebenen Positionen jeweils die Versorgungsspannung zum Bus (4) messen und in einem Speicher des jeweiligen Slavemoduls (2s) gemeinsam mit der Identifizierungsinformation des jeweiligen Slavemoduls (2s) abspeichern, wobei die Identifizierungsanfrage des Mastermoduls (2m) einen vorgegebenen Spannungsbereich enthält und nach Eingang der Identifizierungsanfrage jedes Slavemodul (2s) die in seinem Speicher gespeicherte Versorgungsspannung mit dem vorgegebenen Spannungsbereich der Identifizierungsanfrage vergleicht und ein Slavemodul (2s) seine Identifizierungsinformation an das Mastermodul (2m) übermittelt, falls die gespeicherte Versorgungsspannung des betreffenden Slavemoduls (2s) innerhalb des vorgegebenen Spannungsbereiches der Identifizierungsanfrage liegt.

4. Verfahren nach Anspruch 2 oder 3, **dadurch gekennzeichnet, dass** die Identifizierungsanfrage des Mastermoduls (2m) an die Slavemodule (2s) in einer anfänglichen Enumerationsphase nach Neuanordnung der Slavemodule (2s) an den vorgegebenen Positionen erfolgt, und nach der Identifizierung und Lokalisierung der Slavemodule (2s) in einer nachfolgenden Betriebsphase die am textilen Träger örtlich lokalisierbare Übertragung von Daten zu Steuerungs-, Auswertungs- oder Anzeigezwecken zwischen den Slavemodulen (2s) und dem Mastermodul (2m) erfolgt.

5. Textiler Träger mit einer Mehrzahl von Mikroprozessormodulen (2), die über den Träger verteilt angeordnet sind und zur Spannungsversorgung und Datenübertragung elektrisch miteinander verbunden sind, wobei Aufnahmen am textilen Träger vorgesehen sind, in denen jeweils ein Mikroprozessormodul (2) austauschbar aufgenommen und mit Anschlussstellen (5) elektrisch kontaktierbar ist, **dadurch gekennzeichnet, dass** eines der Mikroprozessormodule (2) als Mastermodul (2m) ausgebildet ist und die verbleibenden Mikroprozessormodule (2) als mit dem Mastermodul (2m) über einen Bus (4) verbundene Slavemodule (2s), wobei jeweils ein Slavemodul (2s) in den Aufnahmen am textilen Träger austauschbar aufgenommen und mit den Anschlussstellen (5) zum Bus (4) elektrisch kontaktierbar ist, und der Bus (4) als ein das Mastermodul (2m) mit den Aufnahmen verbindendes und im textilen Träger verlaufendes, elektrisch leitfähiges Garn ausgeführt ist.

6. Textiler Träger nach Anspruch 5, **dadurch gekennzeichnet, dass** die Aufnahmen als Aufnahmetaschen (1) und die Slavemodule (2s) als flächige Gebilde ausgeführt sind, wobei ein Slavemodul (2s) in eine Aufnahmetasche (1) entlang einer Einschieberichtung einschiebbar und in der Aufnahmetasche (1) lösbar befestigbar ist.

7. Textiler Träger nach Anspruch 5 oder 6, **dadurch gekennzeichnet, dass** der Bus (4) eine erste elektrische Leitung zur Spannungsversorgung (VDD) und eine zweite elektrische Leitung als Masse (GND) umfasst, sowie eine dritte elektrische Leitung für den Empfang von Daten (RXD) und eine vierte elektrische Leitung für das Senden von Daten (TXD).

8. Textiler Träger nach Anspruch 6 und 7, **dadurch gekennzeichnet, dass** für die vier elektrischen Leitungen (VDD, GND, RXD, TXD) vier Druckknöpfe mit einander jeweils zugeordneten Druckknopfteilen an jeder Aufnahmetasche (1) und an jedem Slavemodul (2s) für die lösbare Befestigung und als Anschlussstellen (5) zum Bus (4) vorgesehen sind, deren Anordnung an jeder Aufnahmetasche (1) ausschließlich bei Drehungen des Slavemoduls (2s) um 0° oder 360° um eine zum einzuschiebenden Slavemodul (2s) senkrechte Achse eine aneinander liegende Lage der Druckknopfteile ermöglicht.

9. Textiler Träger nach Anspruch 6 und 7, **dadurch gekennzeichnet, dass** die Anschlussstellen (5) zum Bus (4) als elektrisch leitende Anschlussflächen ausgeführt sind, die auf einander zugewandten Innenflächen der Aufnahmetaschen (1) angeordnet sind und jeweils an der Oberfläche der Slavemodule (2s) vorgesehene Anschlussflächen eines eingeschobenen Slavemoduls (2s) durch Aneinanderliegen elektrisch kontaktieren.

10. Textiler Träger nach Anspruch 9, **dadurch gekennzeichnet, dass** die Anschlussflächen an den einander zugewandten Innenflächen einer Aufnahmetasche (1) bei fehlendem Slavemodul (2s) als durch Aneinanderliegen miteinander kontaktierbare Flächen ausgeführt sind.

11. Textiler Träger nach Anspruch 9 oder 10, **dadurch gekennzeichnet, dass** die Anschlussflächen der Aufnahmetaschen (1) parallel zueinander und senkrecht zur Einschieberichtung der jeweiligen Aufnahmetasche (1) verlaufen und jeweils parallel zueinander an der Oberfläche der Slavemodule (2s) verlaufenden Anschlussflächen der Slavemodule (2s) zugeordnet sind, deren Anordnung ausschließlich bei Drehungen des einzuschiebenden Slavemoduls (2s) um 0° oder 180° um eine zum Slavemodul (2s) senkrechte Achse eine aneinander liegende Lage der Anschlussflächen der Aufnahmetasche (1) und des eingeschobenen Slavemoduls (2s) ermöglicht.

12. Textiler Träger nach einem der Ansprüche 5 bis 11, **dadurch gekennzeichnet, dass** die Slavemodule (2s) mit Sensoren, Aktoren, Eingabeelementen oder Anzeigeeinrichtungen versehen sind.

## Claims

1. Method for identifying and locating microprocessor modules (2) which are arranged distributed on a textile carrier and are electrically interconnected for voltage supply and data transmission, **characterized in that** one of the microprocessor modules (2) is operated as a master module (2m) and the remaining microprocessor modules (2) as slave modules (2s) which are arranged interchangeably at predetermined positions of the textile carrier and are connected to the master module (2m) via a bus (4), wherein for each slave module (2s) the supply voltage between the slave module (2s) and the bus (4) designed as an electrically conductive yarn is measured and the master module (2m) assigns each slave module (2s) to one of the predetermined positions in a localizing manner on the textile carrier by means of the supply voltage which decreases with increasing distance of a slave module (2s) from the master module (2m).

2. Method according to claim 1, **characterized in that** an identification request of the master module (2m) is made to the slave modules (2s), with which the master module (2m) assigns identification information identifying the respective slave module (2s) to a supply voltage measured by a slave module (2s),

3. Method according to claim 2, **characterized in that,** after rearrangement of the slave modules (2s), the slave modules (2s) each measure the supply voltage to the bus (4) at the predetermined positions and store it in a memory of the respective slave module (2s) together with the identification information of the respective slave module (2s), wherein the identification request of the master module (2m) contains a predetermined voltage range and, after receipt of the identification request, each slave module (2s) compares the supply voltage stored in its memory with the predetermined voltage range of the identification request and a slave module (2s) transmits its identification information to the master module (2m) if the stored supply voltage of the respective slave module (2s) is within the predetermined voltage range of the identification request.

4. Method according to claim 2 or 3, **characterized in that** the identification request of the master module (2m) to the slave modules (2s) takes place in an initial enumeration phase after rearrangement of the slave modules (2s) at the predetermined positions, and after the identification and localization of the slave modules (2s) in a subsequent operating phase, the transmission of data for control, evaluation or display purposes between the slave modules (2s) and the master module (2m), which can be localized on the textile carrier, takes place.

5. Textile carrier having a plurality of microprocessor modules (2) which are arranged distributed over the carrier and are electrically interconnected for voltage supply and data transmission, wherein receptacles are provided on the textile carrier, in each of which receptacles a microprocessor module (2) is interchangeably accommodated and can be electrically contacted with connection points (5), **characterized in that** one of the microprocessor modules (2) is designed as a master module (2m) and the remaining microprocessor modules (2) are designed as slave modules (2s) connected to the master module (2m) via a bus (4), wherein a respective slave module (2s) is interchangeably accommodated in the receptacles on the textile carrier and can be electrically contacted with the connection points (5) to the bus (4), and the bus (4) is designed as an electrically conductive yarn connecting the master module (2m) to the receptacles and extending in the textile carrier.

6. Textile carrier according to claim 5, **characterized in that** the receptacles are in the form of receiving pockets (1) and the slave modules (2s) are designed as flat structures, wherein a slave module (2s) can be inserted into a receiving pocket (1) along an insertion direction and can be detachably fastened in the receiving pocket (1).

7. Textile carrier according to claim 5 or 6, **characterized in that** the bus (4) comprises a first electrical line for power supply (VDD) and a second electrical line as ground (GND), as well as a third electrical line for receiving data (RXD) and a fourth electrical line for transmitting data (TXD).

8. Textile carrier according to claims 6 and 7, **characterized in that** for the four electrical lines (VDD, GND, RXD, TXD) four pushbuttons with pushbutton parts assigned to each other are provided on each receiving pocket (1) and on each slave module (2s) for detachable fastening and as connection points (5) to the bus (4), the arrangement of which on each receiving pocket (1) enables the pushbutton parts to be positioned adjacent to one another exclusively when the slave module (2s) is rotated by 0° or 360° about an axis perpendicular to the slave module (2s) to be inserted.

9. Textile carrier according to claims 6 and 7, **characterized in that** the connection points (5) to the bus (4) are designed as electrically conductive connecting surfaces which are arranged on mutually facing inner surfaces of the receiving pockets (1) and electrically contact connecting surfaces of an inserted slave module (2s) provided on the surface of the slave modules (2s) in each case by lying against one another.

10. Textile carrier according to claim 9, **characterized in that** the connecting surfaces on the mutually facing inner surfaces of a receiving pocket (1) are designed as surfaces which can be contacted with one another by lying against one another in the absence of a slave module (2s),

11. Textile carrier according to claim 9 or 10, **characterized in that** the connecting surfaces of the receiving pockets (1) extend parallel to one another and perpendicular to the insertion direction of the respective receiving pocket (1) and are assigned connecting surfaces of the slave modules (2s) which extend parallel to one another on the surface of the slave modules (2s), the arrangement of which enables the connecting surfaces of the receiving pocket (1) and the inserted slave module (2s) to be positioned adjacent to one another only when the slave module (2s) to be inserted is rotated by 0° or 180° about an axis perpendicular to the slave module (2s).

12. Textile carrier according to one of claims 5 to 11, **characterized in that** the slave modules (2s) are provided with sensors, actuators, input elements or display devices.

## Revendications

1. Procédé pour l'identification et la localisation de modules de microprocesseur (2) répartis sur un support textile et reliés électriquement les uns aux autres en vue de l'alimentation électrique et de la transmission de données, **caractérisé en ce que** l'un des modules de microprocesseur (2) est piloté comme un module maître (2m) et les autres modules de microprocesseur (2) comme des modules esclaves (2s) disposés de façon interchangeable en des positions prédéterminées du support textile et reliés au module maître (2m) par un bus (4), la tension d'alimentation étant mesurée pour chaque module esclave (2s) entre le module esclave (2s) et le bus (4) réalisé comme un fil textile conducteur et le module maître (2m) associant chaque module esclave (2s) à l'une des positions prédéterminées sur le support textile pour le localiser à l'aide de la tension d'alimentation qui diminue avec la distance croissante entre un module esclave (2s) et le module maître (2m).

2. Procédé selon la revendication 1, **caractérisé en ce que** le module maître (2m) envoie aux modules esclaves (2s) une interrogation d'identification avec laquelle le module maître (2m) associe à une tension d'alimentation mesurée par un module esclave (2s) une information d'identification identifiant le module esclave (2s) en question.

3. Procédé selon la revendication 2, **caractérisé en ce que**, après une redistribution des modules esclaves (2s), les modules esclaves (2s) mesurent leur tension d'alimentation respective par rapport au bus (4) dans les positions déterminées et enregistrent celle-ci dans une mémoire de chaque module esclave (2s) en même temps que l'identification d'information du module esclave (2s) en question, l'interrogation d'identification du module maître (2m) contenant une plage de tension prédéterminée et chaque module esclave (2s) comparant la tension d'alimentation enregistrée dans sa mémoire avec la plage de tension prédéterminée de l'interrogation d'identification après avoir reçu l'interrogation d'identification et un module esclave (2s) transmettant ses informations d'identification au module maître (2m) si la tension d'alimentation en mémoire du module esclave (2s) en question se situe dans la plage de tension prédéterminée de l'interrogation d'identification.

4. Procédé selon la revendication 2 ou 3, **caractérisé en ce que** l'interrogation d'identification du module maître (2m) est transmise aux modules esclaves (2s) dans une phase initiale d'énumération après la redistribution des modules esclaves (2s) dans les positions déterminées et l'identification et la localisation des modules esclaves (2s) sont suivies, au cours d'une phase de fonctionnement suivante, par la transmission de données localisable dans l'espace sur le support textile de données entre les modules esclaves (2s) et le module maître (2m) aux fins de commande, d'analyse ou d'affichage.

5. Support textile muni de multiples modules de microprocesseur (2) qui sont répartis sur le support et reliés électriquement les uns aux autres en vue de l'alimentation électrique et de la transmission de données, dans lequel sont prévus sur le support textile des logements dans chacun desquels un module de microprocesseur (2) est logé de façon interchangeable et peut être mis en contact électrique avec des points de connexion (5), **caractérisé en ce que** l'un des modules de microprocesseur (2) est conformé comme un module maître (2m) et les modules de microprocesseur (2) restants comme des modules esclaves (2s) reliés au module maître (2m) par un bus (4), chaque module esclave (2s) étant logé de façon interchangeable dans les logements du support textile et pouvant être mis en contact électrique avec les points de connexion (5) vers le bus (4), et le bus (4) est réalisé comme un fil textile conducteur électrique qui relie le module maître (2m) avec les logements et s'étend dans le support textile.

6. Support textile selon la revendication 5, **caractérisé en ce que** les logements sont conformés comme des poches de logement (1) et les modules esclaves (2s) comme des structures planes, un module esclave (2s) pouvant être inséré dans une poche de logement (1) dans une direction d'insertion et fixé dans la poche de logement (1) d'une façon qui peut être défaite.

7. Support textile selon la revendication 5 ou 6, **caractérisé en ce que** le bus (4) comprend une première ligne électrique pour l'alimentation électrique (VDD) et une deuxième ligne électrique pour la masse (GND), ainsi qu'une troisième ligne électrique pour la réception de données (RXD) et une quatrième ligne électrique pour l'émission de données (TXD).

8. Support textile selon les revendication 6 et 7, **caractérisé en ce que** sont prévus pour les quatre lignes électriques (VDD, GND, RXD, TXD) quatre boutons-poussoirs avec des parties de bouton-poussoir associées les unes aux autres sur chaque poche de logement (1) et sur chaque module esclave (2s) pour la fixation pouvant être défaite et pour servir de points de connexion (5) avec le bus (4), dont la disposition sur chaque poche de logement (1) ne permet de placer des parties de bouton-poussoir l'une sur l'autre que quand le module esclave (2s) est tourné de 0° ou 360° autour d'un axe perpendiculaire au module esclave (2s) à insérer.

9. Support textile selon les revendications 6 et 7, **caractérisé en ce que** les points de connexion (5) avec le bus (4) sont réalisés comme des surfaces de connexion conductrices électriques, qui sont disposées sur des faces intérieures des poches de logement (1) tournées l'une vers l'autre et mettent en contact électrique en les appliquant l'une sur l'autre des surfaces de connexion d'un module esclave (2s) inséré prévues sur la surface des modules esclaves (2s).

10. Support textile selon la revendication 9, **caractérisé en ce que** les surfaces de connexion sur les faces intérieures tournées l'une vers l'autre d'une poche de logement (1) sont réalisées, en l'absence d'un module esclave (2s), comme des surfaces pouvant être mises en contact quand elles sont appliquées l'une sur l'autre.

11. Support textile selon la revendication 9 ou 10, **caractérisé en ce que** les surfaces de connexion des poches de logement (1) sont orientées parallèlement l'une à l'autre et perpendiculairement au sens d'insertion de la poche de logement (1) en question et sont associées à des surfaces de connexion des modules esclaves (2s) orientées parallèlement l'une à l'autre sur la surface des modules esclaves (2s), dont la disposition ne permet d'appliquer les surfaces de connexion de la poche de logement (1) et du module esclave (2s) inséré l'une sur l'autre que lorsque le module esclave (2s) est tourné de 0° ou 180° autour d'un axe perpendiculaire au module esclave (2s).

12. Support textile selon l'une des revendications 5 à 11, **caractérisé en ce que** les modules esclaves (2s) sont munis de capteurs, d'actionneurs, d'éléments de saisie ou de dispositifs d'affichage.
